Europäisches Patentamt

**European Patent Office**

**Office européen des brevets**

(19)

(11) Numéro de publication: **0 280 606 B1**

(12) **FASCICULE DE BREVET EUROPEEN**

(45) Date de publication de fascicule du brevet: **15.04.92**

(51) Int. Cl.5: **C09K 15/12**, A61K 7/48, A61K 7/40

(21) Numéro de dépôt: **88400283.3**

(22) Date de dépôt: **08.02.88**

(54) **Nouveau système anti-oxydant à base d'un ester d'ascorbyle stabilisé, contenant en association au moins un agent complexant et au moins un thiol, et compositions contenant un tel système anti-oxydant.**

(30) Priorité: **09.02.87 FR 8701539**

(43) Date de publication de la demande:
**31.08.88 Bulletin 88/35**

(45) Mention de la délivrance du brevet:
**15.04.92 Bulletin 92/16**

(84) Etats contractants désignés:
**BE CH DE FR GB IT LI NL**

(56) Documents cités:
**DE-A- 2 550 648**
**DE-B- 1 239 063**
**FR-A- 1 438 158**
**FR-A- 2 092 822**
**FR-A- 2 282 266**

(73) Titulaire: **L'OREAL**
**14, Rue Royale**
**F-75008 Paris(FR)**

(72) Inventeur: **n'Guyen, Ouang Lan**
**45, avenue Alsace Lorraine**
**F-92160 Antony(FR)**
Inventeur: **Griat, Jacqueline**
**11, Ouai de la Baronnie**
**F-94480 Ablon(FR)**
Inventeur: **Millecamps, François**
**11, Square A. Renoir**
**F-75014 Paris(FR)**

(74) Mandataire: **Nony, Michel et al**
**Cabinet NONY & CIE, 29, rue Cambacérès**
**F-75008 Paris(FR)**

Rank Xerox (UK) Business Services

## Description

La présente invention a pour objet un nouveau système anti-oxydant à base d'un ester d'ascorbyle stabilisé, contenant, en association, au moins un agent complexant et au moins un thiol, l'utilisation d'un tel système anti-oxydant et des compositions à base de matières oléagineuses contenant un tel système, notamment des compositions cosmétiques.

On sait que les corps gras ont tendance à s'oxyder, même à température ambiante, et cette oxydation (ou rancissement) leur fait acquérir de nouvelles propriétés, notamment gustatives et olfactives qui sont généralement considérées comme indésirables lorsque ces corps gras sont incorporés par exemple dans des compositions alimentaires ou dans des compositions cosmétiques.

On utilise donc couramment, dans des compositions contenant des corps gras, des agents protecteurs qui jouent en fait le rôle d'anti-oxydants.

Parmi les anti-oxydants connus, on utilise couramment l'acide ascorbique qui agit notamment par absorption directe d'oxygène. Toutefois, l'acide ascorbique est très peu soluble dans les corps gras et est donc difficilement utilisable pour protéger ceux-ci contre l'oxydation.

Afin de solubiliser la molécule d'acide ascorbique dans les matières grasses, on a proposé l'utilisation de divers esters d'ascorbyle tels que par exemple le stéarate, le palmitate ou le laurate d'ascorbyle ; voir par exemple l'article de C.F. BOURGEOIS, "Revue Française des Corps Gras", n° 9, pages 353-356 (Septembre 1981).

On sait qu'en dehors de leurs propriétés anti-oxydantes propres, les dérivés ascorbiques ont également la propriété d'améliorer l'activité d'agents anti-oxydants tels que les tocophérols ou l'acide caféique et ses esters, en favorisant la régénération de ces agents anti-oxydants ; voir par exemple H.S. OLCOTT, "Oil Soap",18, (1941), 77, le brevet U.S. 2.462.663, ainsi que la demande de brevet français n° 2.282.266.

La demande de brevet français n° 2.282.266 décrit plus particulièrement comme agent anti-oxydant binaire l'association de l'α -tocophérol et du palmitate d'ascorbyle, ce dernier étant décrit comme fixateur de métaux. D'autres fixateurs de métaux sont décrits dans le livre intitulé "Bailey's Industrial Oil and Fat Products" volume 3, page 303 édité par John Wiley & Sons. Parmi ceux-ci, il est fait mention des thiols tels que l'acide carboxyméthyl mercaptosuccinique et l'acide thiodisuccinique.

On a également proposé diverses améliorations de ces agents anti-oxydants binaires, du type dérivés ascorbiques + tocophérols ou dérivés ascorbiques + dérivés caféiques, en prévoyant l'addition d'un troisième constituant améliorant encore les effets anti-oxydants. Parmi les troisièmes constituants de ces systèmes ternaires, on peut citer notamment l'acide p-aminobenzoïque (brevet U.S. 2.462.663), des phospholipides (R.W. RIEMENSCHNEIDER et ai., "Oil Soap" (1944), 47) des amines (KLAUI, "The Functional (Technical) Uses of Vitamins", ed. by M. STEIN, University of Notthingam Seminar Vitamins, London, England, (1971), page 110), etc...

On a maintenant découvert qu'il est possible d'améliorer notablement les propriétés anti-oxydantes des esters d'ascorbyle en utilisant ces anti-oxydants conjointement avec au moins un agent complexant et ou moins un thiol. On observe alors un effet de synergie important.

La présente invention a donc pour objet un nouveau système anti-oxydant à base d'au moins un ester d'ascorbyle stabilisé, caractérisé par le fait, qu'il comprend au moins un agent complexant et au moins un thiol.

L'ester d'ascorbyle est bien entendu un ester soluble dans les corps gras, et en particulier un ester d'un acide aliphatique ayant de 6 à 24 atomes de carbone tel que par exemple le stéarate, le palmitate ou le laurate d'ascorbyle.

On entend par agent complexant un composé qui est capable d'inhiber par chélation l'effet catalytique des métaux de transition à l'état libre dans le milieu.

Parmi les agents complexants utilisables, on citera notamment l'acide éthylènediamine tétracétique (EDTA), le sel pentasodique de l'acide diéthylènetriamine pentacétique, le salicylate d'hexadécylamine (SHDA) l'acide citrique, l'acide tartrique et son sel de sodium, l'acide phytique, le dibenzyldithiocarbamate ou leurs mélanges.

Outre au moins un de ces agents complexants, la compositon de l'invention peut également contenir un agent complexant secondaire comme le sorbitol.

Selon la présente demande, on entend par thiol, un composé réducteur qui contribue à maintenir les esters d'ascorbyle sous leur forme réduite.

Parmi les thiols utilisables selon l'invention, on citera en particulier : la N-acétyl cystéine, le glutathion ou leurs mélanges.

Le nouveau système anti-oxydant tel que défini ci-dessus peut se présenter notamment sous la forme d'une composition liquide huileuse ; ou encore le système anti-oxydant se présente sous la forme d'une solution alcoolique telle qu'éthanolique.

Dans le système anti-oxydant de l'invention, les proportions relatives des trois catégories de constituants principaux dépendent en particulier des masses moléculaires de l'agent complexant et du thiol. Généralement, le thiol est présent à raison de 1 à 37,5 % et l'agent complexant à raison de 2 à 25 % en poids par rapport au poids total de ces trois types de constituants.

Lorsqu'un agent complexant secondaire est utilisé en même temps que l'agent complexant principal, il est présent en une proportion telle que le mélange soit compris entre 2 et 25 % en poids.

L'ester d'ascorbyle est généralement présent dans le système anti-oxydant à trois constituants, en une proportion comprise entre 5 et 87,5 % en poids.

L'invention a également pour objet un système anti-oxydant tel que défini ci-dessus qui contient en outre un autre anti-oxydant choisi parmi les tocophérols et l'acide caféique (ou acide 3,4- dihydroxycinnamique) ou ses esters.

Par "tocophérols", on entend non seulement 1'$\alpha$-tocophérol mais également le $\beta$, $\gamma$ ou $\delta$ tocophérol ainsi que les mélanges de ceux-ci.

Parmi les esters de l'acide caféïque, on peut notamment citer les alkyl esters tel que les méthyl, éthyl ou butyl esters et le phytol ester.

On a constaté de façon tout-à-fait surprenante que, dans de telles associations, l'activité anti-oxydante des tocophérols et de l'acide caféique, résultait d'un effet de synergie important grâce à la présence de l'ester d'ascorbyle stabilisé par le couple agent complexant - thiol.

Selon cette forme de réalisation de l'invention, le système anti-oxydant est de préférence constitué de :
0,5 à 20 % de tocophérol (s) ou d'acide caféique (ou d'un de ses esters)
8 à 70 % d'un ester d'ascorbyle
4 à 20 % d'un agent complexant
2 à 30 % d'un thiol.

Le rapport molaire de l'ester d'ascorbyle au(x) tocophérol(s) ou à l'acide caféique ou un de ses esters doit être de préférence supérieur ou égal à 3.

Cet effet de synergie peut encore être amélioré lorsque le système anti-oxydant est à base de tocophérol (s) en y associant un polypeptide.

Selon cette autre forme de réalisation, la teneur en polypeptide est de préférence comprise entre 1,5 et 80 %.

Le rapport en poids du polypeptide au (x) tocophérol(s) doit être de préférence supérieure ou égal à 3.

Les polypeptides ont un poids moléculaire moyen compris entre environ 1.000 et environ 100.000. Parmi ceux-ci peut en particulier mentionner les suivants :

(a) Le polypeptide vendu sous la dénomination de "KERASOL" (polypeptide de la kératine soluble de poids moléculaire moyen d'environ 100.000) par la Société CRODA Chemicals Ltd.

(b) Le polypeptide vendu sous la dénomination de "Polypeptide SF" (polypeptide de collagène animal partiellement neutralisé de poids moléculaire moyen d'environ 1000) par la Société NAARDEN.

(c) Le polypeptide vendu sous la dénomination de "Polypeptdie LSN" (polypeptide de collagène animal sous forme de sel d'ammonium contenant environ 3 % (max) de sel inorganique) par la Société NAARDEN, et

(d) Le polypeptide vendu sous la dénomination de "LACTOLAN" (polypeptide obtenu à partir du lait frais de vache préalablement délipidé) par les LABORATOIRES SEROBIOLOGIQUES de NANCY.

L'invention a également pour objet des compositions contenant des corps gras, caractérisées par le fait qu'elles renferment au moins un système anti-oxydant tel que défini précédemment.

Les compositions de l'invention peuvent être notamment des compositions alimentaires (huiles comestibles, saindoux, beurre, margarine ou autres succédanés de beurre) ou des compositions cosmétiques.

Les corps gras présents dans les compositions cosmétiques de l'invention sont par exemple des corps gras d'origine animale tels que la cétine (blanc de baleine), la cire d'abeille, la lanoline, le perhydrosqualène, l'huile de tortue, etc... ; des corps gras végétaux sous forme d'huiles, de graisses ou de cires tels que l'huile d'amande douce, l'huile d'avocat, l'huile d'olive,... ; les huiles de coprah ou de palmiste hydrogénées, le beurre de cacao, la cire de Carnauba, la cire de Montana ; ainsi que des huiles synthétiques constituées par des esters et/ou éthers de glycérol ou de glycol tels que par exemple ceux qui sont décrits dans les brevets français n° 75.24656, 75.24657 et 75.24658.

En plus des corps gras plus ou moins oxydables, les compositions cosmétiques peuvent contenir des produits sensibles à l'oxydation tels que par exemple de la vitamine F ou du $\beta$-carotène.

Les compositions cosmétiques selon l'invention se présentent sous la forme de solutions huileuses, d'émulsions, de produits solides ou de lotions. Elles constituent notamment des laits pour les soins de la peau, des crèmes (crèmes pour le visage, pour les mains, pour le corps, crèmes anti-solaires, crèmes démaquillantes, crèmes fonds de teint), des fonds de teint fluides, des laits démaquillants, des laits anti-solaires, des huiles pour le bain, des rouges à lèvres, des fards à paupières, des sticks déordorants, etc...

Selon une forme de réalisation préférée, les compositions cosmétiques se présentent sous forme de crèmes destinées à la protection de l'oxydation des lipides de la peau.

Dans les compositions cosmétiques selon l'invention, le système anti-oxydant tel que défini ci-dessus est généralement présent de sorte que l'on ait les proportions suivantes par rapport au poids total de la composition :

Tocophérol(s)

ou

Acide caféique (ou un de ses esters)     0 à 0,5 %

et de préférence de    0,05 à 0,5 %

Ester d'ascorbyle    0,45 à 1,6 %

Agent complexant    0,2 à 0,5 %

Thiol    0,1 à 0,7 %

Lorsque le système anti-oxydant est à base de tocophérol(s) la proportion du polypeptide éventuellement présent est alors comprise entre 0,05 à 8 % par rapport au poids total de la composition.

Les compositions de l'invention peuvent en outre contenir des composés actifs ou des ingrédients utilisés de façon usuelle dans les compositions mentionnées ci-dessus, tels que des agents tensio-actifs, des colorants, des parfums, des produits astringents, des produits absorbant l'ultra-violet, des solvants organiques, de l'eau, etc...

Ces compositions sont préparées selon les méthodes usuelles.

On va maintenant donner à titre d'illustration plusieurs exemples de systèmes anti-oxydants selon l'invention ainsi que des exemples de compositions cosmétiques contenant de tels systèmes anti-oxydants.

Exemple 1

Palmitate d'ascorbyle    76 %
Acide citrique    16 %
N-acétyl cystéine    8 %

Exemple 2

Palmitate d'ascorbyle    73 %
Acide citrique    9 %
Glutathion    9 %
N-acétyl cystéine    9 %

Exemple 3

Palmitate d'ascorbyle    79 %
Acide citrique    5 %
EDTA    12 %
N-acétyl cystéine    4 %

Exemple 4

Tocophérols (mélange $\alpha,\beta,\gamma$ et $\delta$)    7 %
Palmitate d'ascorbyle    65 %
Acide citrique    7 %
EDTA    13 %
N-acétyl cystéine    8 %

Exemple 5

Tocophérols    11 %
Palmitate d'ascorbyle    65 %
Salicylate d'hexadécylamine    6 %
Sorbitol    9 %

N-acétyl cystéine 9 %

Exemple 6

Tocophérols 16 %
Palmitate d'ascorbyle 63 %
Glutathion 6 %
N-acétyl cystéine 3 %
Acide citrique 3 %
EDTA 9 %

Exemple 7

Acide caféique 8 %
Palmitate d'ascorbyle 49 %
Acide citrique 4 %
EDTA 10 %
Glutathion 10 %
N-acétyl cystéine 19 %

Exemple 8

Acide caféique 7 %
Palmitate d'ascorbyle 65 %
Tartrate de sodium 8 %
N-acétyl cystéine 20 %

| Exemple 9 | |
|---|---|
| Acide caféique | 8 % |
| Palmitate d'ascorbyle | 70 % |
| Salicylate d'hexadécylamine | 15 % |
| N-acétyl cystéine | 7 % |

| Exemple 10 | |
|---|---|
| Tocophérols | 9 % |
| Palmitate d'ascorbyle | 36 % |
| Acide citrique | 4,5 % |
| N-acétyl cystéine | 4,5 % |
| EDTA | 1,5 % |
| Polypeptide "KERASOL" (matière active) | 44,5 % |

| Exemple 11 | |
|---|---|
| Tocophérols | 5 % |
| Palmitate d'ascorbyle | 21,5 % |
| Acide citrique | 2,5 % |
| N-acétyl cystéine | 1 % |
| Glutathion | 1 % |
| EDTA | 2,5 % |
| Polypeptide "Polypeptide SF" (matière active) | 66,5 % |

EXEMPLES DE COMPOSITIONS COSMETIQUES

| I - Crème de soin "émulsion eau-dans-l'huile" | |
|---|---|
| - Lanolate de magnésium | 14,4 % |
| - Alcool de lanoline | 3,6 % |
| - Huile de tournesol | 40,0 % |
| - Myristate d'isopropyle | 8,0 % |
| - Ozokérite | 4,0 % |
| - Vitamine F | 2,0 % |
| - Acide ascorbique | 0,5 % |
| - Lécithine de soja | 5 % |
| - Tocophérols | 0,25 % |
| - Palmitate d'ascorbyle | 1,0 % |
| - Glutathion | 0,1 % |
| - N-acétyl-cystéine | 0,05 % |
| - Acide citrique | 0,05 % |
| - EDTA | 0,15 % |
| - Parfum | 0,8 % |
| - Parahydroxybenzoate de méthyle | 0,3 % |
| - Eau                                q.s.p. | 100 % en poids |

| II - Baume anhydre | |
|---|---|
| - Huile de Karité | 60,0 % |
| - Huile de tournesol | 20,0 % |
| - Vitamine F | 2,0 % |
| - Lécithine de soja | 4,9 % |
| - Tocophérols | 0,2 % |
| - Palmitate d'ascorbyle | 1,13 % |
| - Glutathion | 0,35 % |
| - Acide citrique | 0,15 % |
| - N-acétyl cystéine | 0,35 % |
| - EDTA | 0,15 % |
| - Vaseline                             q.s.p. | 100 % en poids |

| III - Huile pour le visage et le corps | |
|---|---|
| - Huile de karité | 2,0 % |
| - Huile de tournesol | 31,8 % |
| - Vitamine F | 2,0 % |
| - Huile de soja | 32,0 % |
| - Tocophérols | 0,1 % |
| - Acide citrique | 0,05 % |
| - Palmitate d'ascorbyle | 1,0 % |
| - N-acétyl cystéine | 0,1 % |
| - EDTA | 0,15 % |
| - Lécithine de soja | 0,1 % |
| - Huile d'arachide                     q.s.p. | 100 % en poids |

| IV - Crème de soin émulsion huile-dans-l'eau | |
|---|---|
| - Monostéarate de sorbitan à 20 moles d'oxyde d'éthylène (Tween 60) | 1,0% |
| - Stéarate de glycérol | 2,0% |
| - Acide stéarique | 1,4% |
| - Triéthanolamine | 0,7% |
| - Alcool cétylique | 0,5% |
| - Huile de tournesol | 15,0% |
| - Vitamine F | 2,0% |
| - Acide ascorbique | 1,0% |
| - Lécithine de soja | 0,6% |
| - Huile de vaseline | 2,4% |
| - Acide caféique | 0,2% |
| - Palmitate d'ascorbyle | 1,5% |
| - Salicylate d'hexadécylamine | 0,5% |
| - N-Acétyl cystéine | 0,3% |
| - Polymère carboxyvinylique vendu sous le nom de "Carbopol 940" par la Société Goodrich | 0,2% |
| - Triéthanolamine | 0,2% |
| - Parfum | 0,8% |
| - Conservateur (Parahydroxybenzoate de méthyle) | 0,25% |
| - Eau                                q.s.p. | 100 % en poids |

## V - Fluide de soin pour le corps

- Lipide amphiphile non ionique de formule générale

$$R - (OCH_2 - \underset{\underset{CH_2OH}{|}}{CH})_n -OH$$

dans laquelle R est un radical hexadécyle et n a une valeur statistique moyenne égale à 3................ 4,5%

- Cholestérol................................ 4,5%

- Dicétylphosphate........................... 1,0%

- Parahydroxybenzoate de méthyle............. 0,3%

- Eau déminéralisée stérile.................. 30,0%

2ème PHASE

On ajoute à la dispersion de sphérules obtenue dans la première phase les substances suivantes :

| | |
|---|---|
| - Parfum | 0,4% |
| - Huile de tournesol | 10,0% |
| - Huile de paraffine | 4,0% |
| - Vitamine F | 2,0% |
| - Lécithine de soja | 1,0% |
| - Acide caféique | 0,1% |
| - Palmitate d'ascorbyle | 1,0% |
| - Salicylate d'hexadécylamine | 0,2% |
| - N-acétyl cystéine | 0,1% |
| - Polymère carboxyvinylique vendu sous le nom de "Carbopol 940" par la Société Goodrich | 0,4% |
| - Triéthanolamine | 0,4% |
| - Eau déminéralisée   q.s.p. | 100 % en poids |

| VI - Crème solaire | |
|---|---|
| - Lanolate de magnésium | 7,2 % |
| - Alcool de lanoline | 1,8 % |
| - Huile de tournesol | 20,6 % |
| - Vitamine F | 2,0 % |
| - $\beta$-carotène | 0,1 % |
| - Lécithine de soja | 0,4 % |
| - Huile de paraffine | 4,0 % |
| - Acide caféique | 0,1 % |
| - Palmitate d'ascorbyle | 0,4 % |
| - Acide citrique | 0,05% |
| - Glutathion | 0,05% |
| - N-acétyl cystéine | 0,05% |
| - EDTA | 0,15% |
| - Acide ascorbique | 1% |
| - Poudre de polyéthylène | 10,0% |
| - Acide 2-phényl benzimidazole 5-sulfonique vendu sous la dénomination de "EUSOLEX 232" par la Société MERCK | 3,0% |
| - 2-hydroxy 4-méthoxy benzophénone vendu sous la dénomination de "UVINUL M40" par la Société BASF | 3,0% |
| - Parfum | 1,0% |
| - Parahydroxybenzoate de méthyle | 0,15% |
| - Parahydroxybenzoate de propyle | 0,15% |
| - Eau                                                 q.s.p. | 100 % en poids |

| VII -Huile pour le visage et le corps | |
|---|---|
| - Huile de karité | 2,0 % |
| - Huile de Tournesol | 31,8 % |
| - Vitamine F | 2,0 % |
| - Huile de soja | 32,0 % |
| - Tocophérols | 0,3 % |
| - Acide citrique | 0,15 % |
| - Palmitate d'ascorbyle | 1,2 % |
| - N-acétyl cystéine | 0,15 % |
| - EDTA | 0,05 % |
| - Polypeptide "KERASOL" (matière active) | 1,5 % |
| - Lécithine de soja | 0,1 % |
| - Huile d'arachide                    q.s.p. | 100 % en poids |

| VIII -Crème de soin "Emulsion eau-dans-l'huile" | |
|---|---|
| - Lanolate de magnésium | 14,4 % |
| - Alcool de lanoline | 3,6 % |
| - Huile de Tournesol | 40,0 % |
| - Myristate d'isopropyle | 8,0 % |
| - Ozokérite | 4,0 % |
| - Vitamine F | 2,0 % |
| - Acide ascorbique | 0,6 % |
| - Lécithine de soja | 5 % |
| - Tocophérols | 0,2 % |
| - Palmitate d'ascorbyle | 0,9 % |
| - Glutathion | 0,05 % |
| - N-acétyl-cystéine | 0,05 % |
| - Acide citrique | 0,1 % |
| - EDTA | 0,1 % |
| - Polypeptide "Polypeptide SF" (matière active) | 2,8 % |
| - Parfum | 0,8 % |
| - Parahydroxybenzoate de méthyle | 0,3 % |
| - Eau                            q.s.p. | 100 % en poids |

1 - Etude sur la stabilisation du palmitate d'ascorbyle

Afin de déterminer l'effet stabilisant du couple agent complexant - thiol sur le palmitate d'ascorbyle, plusieurs solutions dans l'éthanol ont été étudiées.

La détermination, dans le temps, du taux de dégradation du palmitate d'ascorbyle a été effectuée par HPLC.

Les solutions étudiées ont été les suivantes :

| Solution A : | Palmitate d'ascorbyle | à 0,05 % |
|---|---|---|
| Solution B : | Palmitate d'ascorbyle | à 0,05 % |
| | N-acétylcystéine | à 0,01 % |
| Solution C : | Palmitate d'ascorbyle | à 0,05 % |
| | EDTA | à 0,01 % |
| Solution D : | Palmitate d'ascorbyle | à 0,05 % |
| | N-acétylcystéine | à 0,01 % |
| | EDTA | à 0,01 % |

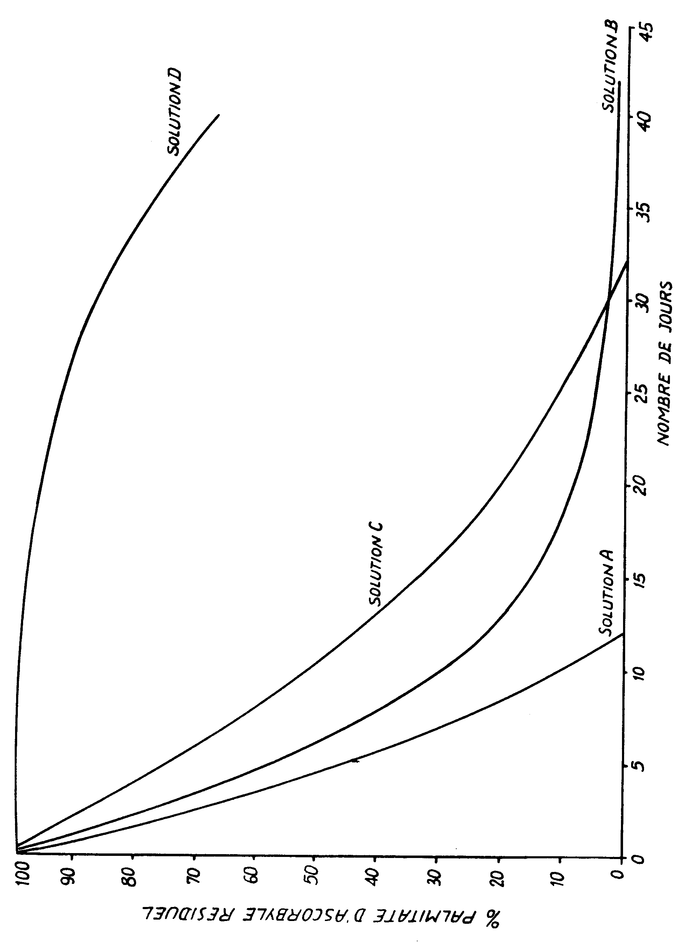

Les résultats obtenus sont reportés sur la figure 1.

Comme on peut le constater en fonction de la courbe correspondant à la solution D, le taux de dégradation du palmitate d'ascorbyle est très nettement inférieur aux autres solutions ce qui démontre le caractère stabilisant du couple agent complexant (EDTA) - thiol (N-acétylcystéine).

En effet, au bout de 40 jours, le taux de dégradation du palmitate d'ascorbyle dans la solution D n'est que de 30% environ alors que pour les solutions A, B et C, le palmitate d'ascorbyle est totalement dégradé au bout de la même période.

2 - Etude de l'effet de synergie entre le palmitate d'ascorbyle, stabilisé par le mélange SHDA + N-acétylcystéine, et les tocophérols

L'huile utilisée dans cet essai est la vitamine F.

On a préparé des mélanges de vitamine F avec diverses quantités de tocophérols, de palmitate d'ascorbyle, de salicylate d'hexadécylamine (SHDA) et de N-acétylcystéine. Les quantités sont précisées dans le tableau I ci-après ; elles sont exprimées en % et sont rapportées à une même quantité (100g) de vitamine F.

On porte les échantillons étudiés à 100°C sous un barbotage d'air (20 1/h) de façon à effectuer une oxydation accélérée de l'huile.

On suit alors en continu la concentration en acides volatils, résultant de la dégradation des hydroperoxydes et des aldéhydes, formés par oxydation, dans une cellule remplie d'eau dans laquelle plonge une electrode de platine. Cette électrode mesure l'évolution de la conductivité en fonction du temps. L'oxydation provoque une augmentation de la conductivité.

Le temps d'induction, qui représente le temps au bout duquel l'oxydation commence, est déterminé par l'intersection des deux tangentes à la courbe d'autooxydation.

TABLEAU I

| Palmitate ascorbyle | SHDA* | N-Acétylcystéine | Tocophérols | Temps d'induction |
|---|---|---|---|---|
| - | - | - | 0,20 | 60 min. |
| 0,20 | - | - | - | 20 min. |
| 0,20 | - | - | 0,20 | 92 min. |
| - | 0,20 | 0,10 | - | 15 min. |
| 0,20 | 0,20 | - | 0,20 | 93 min. |
| 0,20 | - | 0,10 | 0,20 | 96 min. |
| 0,20 | 0,20 | 0,10 | 0,20 | 114 min. |

* salicylate d'hexadécylamine

Le temps d'induction de la vitamine F, seul, sans agent anti-oxydant est de 15 minutes.

Le tableau I montre que l'association du salicylate d'hexadécylamine et de N-acétyl cystéine, seule, ne produit aucun effet. Par contre, comme cela est connu, l'association alpha-tocophérol + palmitate d'ascorbyle amène une augmentation importante du temps d'induction. Ce temps d'induction est encore notablement amélioré par l'association salicylate d'hexadécylamine + N-acétyl cystéine alors que chacun de ces deux agents ajouté seul au mélange alpha-tocophérol + palmitate d'ascorbyle est pratiquement sans influence.

3 - Etude de l'effet de synergie entre le palmitate d'ascorbyle, stabilisé par le mélange SA + N-acétylcystéine et l'acide caféique

On opère comme précédemment. Les résultats sont rassemblés dans le tableau II.

EP 0 280 606 B1

TABLEAU II

| Palmitate d'ascorbyle | SHDA | N-acétylcystéine | Acide caféique | Temps d'induction |
|---|---|---|---|---|
| 0,25 | - | - | 0,10 | 93 min. |
| 0,21 | 0,21 | 0,10 | 0,10 | 315 min. |

Le tableau II montre l'effet de synergie apporté par l'addition, au système étudié, de l'association salicylate d'hexadécylamine - N-acétyl cystéine.

Cette association augmente considérablement le temps d'induction du système acide caféique + palmitate d'ascorbyle, alors qu'utilisée seule, cette association, comme cela a été montré dans le tableau I ci-dessus n'a aucun effet.

**Revendications**

1.  Système anti-oxydant ayant un effet de synergie, caractérisé par le fait qu'il est constitué de l'association d'un ester d'ascorbyle stabilisé, d'un agent complexant et d'un thiol, ledit agent complexant étant choisi parmi l'acide éthylènediamine tétracétique, le sel pentasodique de l'acide diéthylènediamine pentacétique, le salicylate d'hexadécylamine, l'acide citrique, l'acide tartrique, le tartrate de sodium, l'acide phytique, le dibenzyldithiocarbamate ou leurs mélanges, et ledit thiol étant choisi parmi la N-acétylcystéine, le glutathion ou leurs mélanges.

2.  Système anti-oxydant selon la revendication 1, caractérisé par le fait que l'ester d'ascorbyle est un ester d'acide aliphatique ayant de 6 à 24 atomes de carbone tels que le stéarate, le palmitate ou le laurate d'ascorbyle ou leurs mélanges.

3.  Système anti-oxydant selon l'une quelconque des revendications précédentes, caractérisé par le fait qu'il contient en outre au moins un agent complexant secondaire tel que le sorbitol.

4.  Système anti-oxydant selon la revendication 1 ou 2, caractérisé par le fait que l'ester d'ascorbyle est présent de 5 à 87,5 % en poids.

5.  Système anti-oxydant selon l'une quelconque des revendications précédentes, caractérisé par le fait que l'agent complexant est présent à raison de 2 à 25 % en poids et que le thiol est présent à raison de 1 à 37,5 % en poids.

6.  Système anti-oxydant selon l'une quelconque des revendications précédentes, caractérisé par le fait qu'il contient en outre au moins un tocophérol ou un mélange de tocophérols.

7.  Système anti-oxydant selon la revendication 6, caractérisé par le fait qu'il contient en outre un polypeptide, le rapport en poids de polypeptides au(x) tocophérol(s) devant être supérieur ou égal à 3.

8.  Système anti-oxydant selon l'une quelconque des revendications 1 à 7, caractérisé par le fait qu'il contient en outre de l'acide caféique ou un de ses esters.

9.  Système anti-oxydant selon la revendication précédente, caractérisé par le fait qu'il est constitué de :
    0,5 à 20 % de tocophérol(s) ou d'acide caféïque (ou d'un de ses esters)
    8 à 70 % d'un ester d'ascorbyle
    4 à 20 % d'un agent complexant
    2 à 30 % d'un thiol.

10.  Système anti-oxydant selon l'une quelconque des revendications 1 à 7 et 9, caractérisé par le fait qu'il est constitué de :
    0,5 à 20 % de tocophérol(s)
    8 à 70 % d'un ester d'ascorbyle
    4 à 20 % d'un agent complexant
    2 à 30 % d'un thiol
    1,5 à 80 % d'un polypeptide.

11

**11.** Système anti-oxydant selon l'une quelconque des revendications précédentes caractérisé par le fait que le rapport molaire de l'ester d'ascorbyle au(x) tocophérol(s) ou à l'acide caféique ou l'un de ses esters est supérieur ou égal à 3.

**12.** Composition contenant des corps gras caractérisée par le fait qu'elle contient un système anti-oxydant selon l'une quelconque des revendications 1 à 11.

**13.** Composition cosmétique caractérisée par le fait qu'elle contient un système anti-oxydant selon l'une quelconque des revendications 1 à 11, les proportions des constituants du système anti-oxydant par rapport au poids total de la composition étant de :

| Tocophérol(s) ou Acide caféique (ou un de ses esters) préférence | 0 à 0,5 % et de 0,05 à 0,5 % |
|---|---|
| Ester d'ascorbyle | 0,45 à 1,6 % |
| Agent complexant | 0,2 à 0,5 % |
| Thiol | 0,1 à 0,7 % |

**14.** Composition cosmétique selon la revendication 13, caractérisée par le fait que les proportions des constituants du système anti-oxydant par rapport au poids total de la composition sont de :

| Tocophérol(s) | 0,05 à 0,5 % |
|---|---|
| Ester d'ascorbyle | 0,45 à 1,6 % |
| Agent complexant | 0,2 à 0,5 % |
| Thiol | 0,1 à 0,7 % |
| Polypeptide | 0,05 à 8 % |

**15.** Composition cosmétique selon l'une quelconque des revendications 13 et 14, caractérisée par le fait que l'ester d'ascorbyle est le palmitate d'ascorbyle.

**16.** Composition cosmétique selon l'une quelconque des revendications 13 à 15, caractérisée par le fait que l'agent complexant est l'acide éthylènediamine tétracétique, le salicylate d'hexadécylamine, l'acide citrique, le sel pentasodique de l'acide diéthylènetriamine pentacétique, l'acide tartrique ou son sel de sodium, l'acide phytique, le dibenzyldithiocarbamate ou un mélange de ceux-ci.

**17.** Composition cosmétique selon l'une quelconque des revendications 13 à 15, caractérisée par le fait que le thiol est la N-acétylcystéine, le glutathion ou leurs mélanges.

**18.** Composition cosmétique selon l'une quelconque des revendications 13 à 17, caractérisée par le fait qu'elle se présente sous forme d'une crème destinée à la protection de l'oxydation des lipides de la peau.

**Claims**

**1.** Antioxidant system having a synergistic action, characterized in that it consists of the combination of a stabilised ascorbyl ester, a complexing agent and a thiol, the said complexing agent being chosen from ethylenediaminetetraacetic acid, the pentasodium salt of diethylenetriaminepentaacetic acid, hexadecylamine salicylate, citric acid, tartaric acid, sodium tartrate, phytic acid, dibenzyl dithiocarbamate or mixtures thereof, and the said thiol being chosen from N-acetylcysteine, glutathione or mixtures thereof.

**2.** Antioxidant system according to claim 1, characterized in that the ascorbyl ester is an ester of an aliphatic acid containing from 6 to 24 carbon atoms, such as ascorbyl stearate, palmitate or laurate or mixtures thereof.

3. Antioxidant system according to any one of the preceding claims, characterized in that it additionally contains at least one secondary complexing agent such as sorbitol.

4. Antioxidant system according to Claim 1 or 2, characterized in that the ascorbyl ester is present in a proportion of 5 to 87.5 % by weight.

5. Antioxidant system according to any one of the preceding claims, characterized in that the complexing agent is present in a proportion of 2 to 25 % by weight and that the thiol is present in a proportion of 1 to 37.5 % by weight.

6. Antioxidant system according to any one of the preceding claims, characterized in that it additionally contains at least one tocopherol or a mixture of tocopherols.

7. Antioxidant system according to Claim 6, characterized in that it additionally contains a polypeptide, the weight ratio of polypeptides to the tocopherol(s) having to be greater than or equal to 3.

8. Antioxidant system according to any one of Claims 1 to 7, characterized in that it additionally contains caffeic acid or one of its esters.

9. Antioxidant system according to the preceding claim, characterized in that it consists of:
   0.5 to 20 % of tocopherol(s) or of caffeic acid (or of one of its esters)
   8 to 70 % of an ascorbyl ester
   4 to 20 % of a complexing agent
   2 to 30 % of a thiol.

10. Antioxidant system according to any one of Claims 1 to 7 and 9, characterized in that it consists of:
    0.5 to 20 % of tocopherol(s)
    8 to 70 % of an ascorbyl ester
    4 to 20 % of a complexing agent
    2 to 30 % of a thiol
    1.5 to 80 % of a polypeptide.

11. Antioxidant system according to any one of the preceding claims, characterized in that the molar ratio of the ascorbyl ester to the tocopherol(s) or to caffeic acid or one of its esters is greater than or equal to 3.

12. Composition containing fatty substances, characterized in that it contains an antioxidant system according to any one of Claims 1 to 11.

13. Cosmetic composition characterized in that it contains an antioxidant system according to any one of Claims 1 to 11, the proportions of the constituents of the antioxidant system relative to total weight of the composition being:

| | |
|---|---|
| Tocopherol(s) or Caffeic acid (or one of its esters) | 0 to 0.5 % |
| and preferably | 0.05 to 0.5 % |
| Ascorbyl ester | 0.43 to 1.6 % |
| Complexing agent | 0.2 to 0.5 % |
| Thiol | 0.1 to 0.7 % |

14. Cosmetic composition according to Claim 13, characterized in that the proportions of the constituents of the antioxidant system relative to the total weight of the composition are:

| Tocopherol(s) | 0.05 to 0.5 % |
|---|---|
| Ascorbyl ester | 0.45 to 1.6 % |
| Complexing agent | 0.2 to 0.5 % |
| Thiol | 0.1 to 0.7 % |
| Polypeptide | 0.05 to 8 % |

15. Cosmetic composition according to either of Claims 13 and 14, characterized in that the ascorbyl ester is ascorbyl palmitate.

16. Cosmetic composition according to any one of Claims 13 to 15, characterized in that the complexing agent is ethylenediaminetetraacetic acid, hexadecylamine salicylate, citric acid, pentasodium salt of diethylenetriaminepentaacetic acid, tartaric acid or its sodium salt, phytic acid, dibenzyl dithiocarbamate or a mixture thereof.

17. Cosmetic composition according to any one of Claims 13 to 15, characterized in that the thiol is N-acetylcysteine, glutathione or mixtures thereof.

18. Cosmetic composition according to any one of Claims 13 to 17, characterized in that it is in the form of a cream intended for protecting the lipids of the skin against oxidation.

**Patentansprüche**

1. Antioxidierendes System mit synergistischem Effekt, **dadurch gekennzeichnet,** daß es aus einer Kombination eines stabilisierten Ascorbylesters, eines komplexbildenden Mittels und eines Thiols besteht, wobei das komplexbildende Mittel ausgewählt ist unter Ethylendiamintetraessigsäure, dem Pentanatriumsalz von Diethylentriaminpentaessigsäure, Hexedecylaminsalicylat, Citronensäure, Weinsäure, Natriumtartrat, Phytinsäure, Dibenzyldithiocarbamat oder Gemischen davon, und wobei das Thiol ausgewählt ist unter N-Acetylcystein, Glutathion oder Gemischen davon.

2. Antioxidierendes System nach Anspruch 1, **dadurch gekennzeichnet,** daß der Ascorbylester ein Ester einer aliphatischer Säure mit 6 bis 24 Kohlenstoffatomen, wie z. B. Ascorbylstearat, -palmitat oder -laurat oder ein Gemisch davon, ist.

3. Antioxidierendes System nach einem der vorhergehenden Ansprüche, **dadurch gekennzeichnet,** daß es außerdem ein sekundäres komplexbildendes Mittel, wie Sorbit, enthält.

4. Antioxidierendes System nach Anspruch 1 oder 2, **dadurch gekennzeichnet,** daß der Ascorbylester in einer Menge von 5 bis 87,5 Gew.-% anwesend ist.

5. Antioxidierendes System nach einem der vorhergehenden Ansprüche, **dadurch gekennzeichnet,** daß das komplexbildende Mittel in einer Menge von 2 bis 25 Gew.-% und das Thiol in einer Menge von 1 bis 37,5 Gew.-% anwesend sind.

6. Antioxidierendes System noch einem der vorhergehenden Ansprüche, **dadurch gekennzeichnet,** daß es außerdem mindestens ein Tocopherol oder eine Mischung von Tocopherolen enthält.

7. Antioxidierendes System nach Anspruch 6, **dadurch gekennzeichnet,** daß es außerdem ein Polypeptid enthält, wobei das Gewichtsverhältnis zwischen den Polypeptiden und dem (den) Tocopherol(en) größer oder gleich 3 sein soll.

8. Antioxidierendes System nach einem der Ansprüche 1 bis 7, **dadurch gekennzeichnet,** daß es außerdem Kaffeesäure oder einen der Ester davon enthält.

9. Antioxidierendes System nach einem der vorhergehenden Ansprüche, **dadurch gekennzeichnet,** daß es zusammengesetzt ist aus:
   0,5 bis 20 % Tocopherol(en) oder Kaffeesäure (oder einem der Ester davon)

8 bis 70 % Ascorbylester
4 bis 20 % komplexbildendes Mittel
2 bis 30 % Thiol

**10.** Antioxidierendes System nach einem der Ansprüche 1 bis 7 und 9, **dadurch gekennzeichnet,** daß es zusammengesetzt ist aus:
0,5 bis 20 % Tocopherol(en)
8 bis 70 % Ascorbylester
4 bis 20 % komplexbildendes Mittel
2 bis 30 % Thiol
1,5 bis 80 % Polypeptid

**11.** Antioxidierendes System nach einem der vorhergehenden Ansprüche, **dadurch gekennzeichnet,** daß das Molverhältnis zwischen Ascorbylester und Tocopherol(en) oder Kaffeesäure oder einem Ester davon größer oder gleich 3 ist.

**12.** Fettkörper enthaltendes Mittel, **dadurch gekennzeichnet,** daß es ein antioxidierendes System nach einem der Ansprüche 1 bis 11 enthält.

**13.** Kosmetisches Mittel, **dadurch gekennzeichnet,** daß es ein antioxidierendes System nach einem der Ansprüche 1 bis 11 enthält, wobei die Komponenten des antioxidierenden Systems in folgender Menge, bezogen auf des Gesamtgewicht des Mittels, enthalten sind:

| | |
|---|---|
| - Tocopherol(e) oder Kaffeesäure (oder ein Ester davon), vorzugsweise | 0 bis 0,5 %<br>0,05 bis 0,5 % |
| - Ascorbylester | 0,45 bis 1,6 % |
| - komplexbildendes Mittel | 0,2 bis 0,5 % |
| - Thiol | 0,1 bis 0,7 % |

**14.** Kosmetisches Mittel nach Anspruch 13, **dadurch gekennzeichnet,** daß die Komponenten des antioxidierenden Systems in folgender Mengen, bezogen auf das Gesamtgewicht des Mittels, enthalten sind:

| | |
|---|---|
| - Tocopherol(e) | 0,05 bis 0,5 % |
| - Ascorbylester | 0,45 bis 1,6 % |
| - komplexbildendes Mittel | 0,2 bis 0,5 % |
| - Thiol | 0,1 bis 0,7 % |
| - Polypeptid | 0,05 bis 8 % |

**15.** Kosmetisches Mittel nach einem der Ansprüche 13 und 14, **dadurch gekennzeichnet,** daß es sich bei dem Ascorbylester um Ascorbylpalmitat handelt.

**16.** Kosmetisches Mittel nach einem der Ansprüche 13 bis 15, **dadurch gekennzeichnet,** daß es sich bei dem komplexbildenden Mittel um Ethylendiamintetraessigsäure, Hexadecylminsalicylat, Citronensäure, das Pentanatriumsalz der Diethylentriaminpentaessigsäure, Weinsäure oder das Natriumsalz davon, Phytinsäure oder Dibenzyldithiocarbamat oder eine Mischung davon handelt.

**17.** Kosmetisches Mittel nach einem der Ansprüche 13 bis 15, **dadurch gekennzeichnet,** daß es sich bei dem Thiol um N-Acetylcystein, Glutathion oder Mischungen davon handelt.

**18.** Kosmetisches Mittel nach einem der Ansprüche 13 bis 17, **dadurch gekennzeichnet,** daß es in Form einer Crème zum Schutz vor Oxidation der Hautlipide vorliegt.